# EUROPEAN PATENT APPLICATION

(11) **EP 3 501 609 A1**
(43) Date of publication of application: **26.06.2019**
(21) Application number: 18020631.0
(22) Date of filing: 07.12.2018
(51) Int. Cl.: A61Q 90/00, A61K 8/99, A61K 45/06, A61K 33/44, A61P 43/00, A61K 31/519

(54) **PHARMACEUTICAL COMPOSITIONS COMPRISING IBRUTINIB**

(30) Priority: 08.12.2017 CZ 20170787
(71) Applicant: Zentiva K.S., 102 37 Praha 10 (CZ)
(72) Inventor: Zvatora, Pavel, 198 00 Praha 9 (CZ); Beranek, Josef, 160 00 Praha 6 (CZ); Cernova, Miroslava, 169 00 Praha 6 (CZ); Tieger, Ester, 9963 Magyarlak (HU); Zvonicek, Vit, 170 00 Praha 7 (CZ)
(74) Representative: Jirotkova, Ivana

(57) **Abstract**

The solution relates to a pharmaceutical composition without surfactants comprising ibrutinib form C of formula and at least one acceptable excipient selected from the group consisting of a filler, binder, disintegrants and lubricants. The composition comprising ibrutinib of form C while this form has higher solubility, and thus comprises no surfactant fraction. The composition may take the form of tablets or capsules.

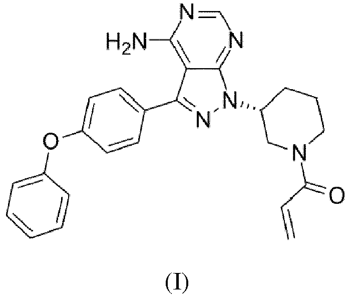

## Description

### Field of the invention

The invention relates to selected pharmaceutical compositions comprising 1-[(3*R*)-3-[4-Amino-3-(4-phenoxyphenyl)-1H-pyrazolo[3,4-d]pyrimidin-1-yl]piperidin-1-yl]prop-2-en-1-one of formula **I,** known as ibrutinib, 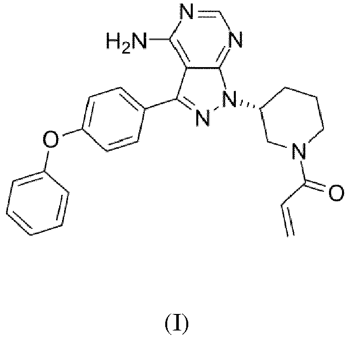 in the free base form (form C). The invention further comprises various preparation methods of pharmaceutical compositions comprising this pharmaceutically active ingredient.

### Background Art

Ibrutinib is, under the commercial name Imbruvica® (140 mg), used in pharmacy for the treatment of adult patients with a relapsing or refractory mantle cell lymphoma (MCL), for the treatment of adult patients with chronic lymphocytic leukaemia (CLL) and for the treatment of adult patients with Waldenstrom macroglobulinemia (WM).

Preparation of ibrutinib and its isolation by means of chromatography was first described in the patent application WO 2008/039218. Preparation of crystalline forms of ibrutinib in a solvated or non-solvated form was subsequently described in the patent applications WO 2013/184572, WO 2015/081180, WO 2015/145415, WO 2016/050422, WO 2016/079216, WO 2016/139588, WO 2016/127960, WO2016/206662, WO 2016/150349, WO 2016/156127, WO 2016/160598, WO 2016/160604, WO 2016/207172, WO 2017/029586.

The solid crystalline form of ibrutinib used in this invention was described in the patent application WO 2017/174044. This form was first mentioned in the patent application WO 2013/184572 as form C.

The use of ibrutinib for the treatment of lymphomas was described in the patent applications WO 2008/039218 and WO 2011/153514. Further patent applications WO 2010/009342 and WO 2015/023703 extend indication of this drug to oncological breast disorders as well. The following patent applications WO 2015/061751 and WO 2015/084857 describe its use in immunosuppressive treatment, treatment of T-cell malignancies (WO 2014/071231), treatment of oncological disorders of the central nervous system (WO 2016/010926), treatment of diffuse non-small cell lymphoma (WO 2016/019341, WO 2016/020901), support of stem cell growth in patients with an oncological disorder (WO 2016/044774), treatment of acute myeloid leukaemia (WO 2016/065674), treatment of oncological disorders of the thyroid gland (WO 2016/090021), and the treatment of Parkinson's disease (WO 2017/072335).

The present invention provides selected pharmaceutical compositions comprising ibrutinib (form C) and methods of their preparation.

### Disclosure of the Invention

The object of the invention provides selected pharmaceutical compositions comprising ibrutinib of form C and preparation methods of these pharmaceutical compositions. Pharmaceutical compositions according to the present invention can be in the form of capsules or tablets with immediate release of the active ingredient, gradual or protracted release of the active ingredient. The formulation process can comprise grinding, mixing, sieving, compaction, dry or wet granulation, tabletting or filling of capsules.

Ibrutinib free base, form A, used in the treatment preparation Imbruvica®, is virtually insoluble in water in the pH range of 4.5-8. At pH 1.2, this substance is poorly soluble. According to the biological classification system, ibrutinib is classified in category II, which comprises substances with low solubility in water and substances with good passage capability through biological membranes (SPC of the preparation Imbruvica® 140 mg hard gelatin capsules).

Due to the low solubility of form A of ibrutinib, within the preparation of Imbruvica®, the solubility of this drug form required optimization. For these reasons, a pilot pharmacokinetic animal study has been conducted to study the influence of micronization of form A of ibrutinib and the contents of various surfactants in the pharmaceutical composition.

Thanks to higher solubility of form C of ibrutinib, there is no need for a surfactant (wetting agent) to be present in pharmaceutical compositions according to the present invention and the size of particles of ibrutinib does not need to be adapted by micronization either. While maintaining the same dose of the active ingredient, the same solubility and thus bioavailability was achieved in a dissolution experiment as in the case of the original composition prepared according to the patent application 2013/184572. Compared to the original preparation Imbruvica®, the absence of a wetting agent in pharmaceutical compositions according to the present invention reduces stomach irritation and the occurrence of some side effects. The presence of the surfactant sodium lauryl sulphate has been surprisingly found to have a negative influence on solubility and thus bioavailability in the case of a formulation comprising form C of ibrutinib.

An object of the invention is a pharmaceutical composition comprising crystalline form C of ibrutinib free base and at least one pharmaceutically acceptable excipient while the pharmaceutical composition does not comprise a surfactant. In some embodiments, the at least one pharmaceutically acceptable excipient is selected from a group that consists of fillers, binders, disintegrants and lubricants. In some embodiments, the pharmaceutical composition may comprise crystalline form C of ibrutinib free base in the quantity of 30 to 50% by weight, filler in the quantity of 20 to 60% by weight and other excipients up to 100% content of the composition, which are a disintegrant, binder and/or lubricant. The pharmaceutical composition may be in the form of a capsule, tablet or coated tablet. The film coating can comprise a binder in the quantity of 50 to 70% by weight, a plasticizer in the quantity of 3 to 14% by weight, a dye in the quantity of 15 to 25% by weight, a pigment in the quantity of 0.1 to 1.0% by weight and a glidant in the quantity of 8.5 to 10% by weight.

Pharmaceutical compositions according to the present invention may be further packed in a protective package, in a protective package with a desiccant, in a protective package with an oxygen absorbent, or in a protective package with a protective inert atmosphere.

Another object of the invention is a pharmaceutical composition comprising:
140 mg of crystalline form C of ibrutinib free base;
165 mg of microcrystalline cellulose;
23 mg of sodium salt of croscarmellose;
2 mg of magnesium stearate.

### Brief description of the Drawings

**Figure 1****:** Dissolution from powder: a) Imbruvica® - ibrutinib (form A); b) mixture of ibrutinib and excipients (form C - according to Example 6) before compaction; c) compaction product of ibrutinib and excipients (form C - according to Example 6), compaction force 10 kN; d) compaction product of ibrutinib and excipients (form C - according to Example 6), compaction force 25 kN
**Figure 2****:** Dissolution from powder: a) Imbruvica® - ibrutinib (form A); b) mixture of ibrutinib and excipients (form C - according to Example 7) before compaction; c) compaction product of ibrutinib and excipients (form C - according to Example 7), compaction force 10 kN; d) compaction product of ibrutinib and excipients (form C - according to Example 7), compaction force 25 kN
**Figure 3****:** Diffractogram of X-ray powder diffraction of crystalline ibrutinib free base (form 1)
**Figure 4****:** Diffractogram of X-ray powder diffraction of crystalline ibrutinib free base (form 2)
**Figure 5****:** Diffractogram of X-ray powder diffraction of crystalline ibrutinib free base (form 3)
**Figure 6****:** Diffractogram of X-ray powder diffraction of crystalline ibrutinib free base (form 4)

### Detailed description of the Invention

The object of the invention provides selected pharmaceutical compositions comprising ibrutinib of form C and preparation methods of these pharmaceutical compositions. The formulation process can comprise grinding, mixing, sieving, compaction, briquetting, dry or wet granulation, tabletting or filling of capsules.

The prepared mixture can be filled into capsules of size 00 or tabletted into round tablets with the diameter of 9 mm. The tablets can be preferably coated with a functional coat protecting the active ingredient from light. The functional coat can comprise at least one excipient from the group of polymers (e.g. hypromellose, hydroxypropyl cellulose, polyvinyl alcohol, methyl cellulose, ethyl cellulose). Plasticizers (e.g. polyethylene glycol, triacetin, triethyl citrate or mineral oil) and other additives as fillers and glidants (e.g. talc, kaolin, lecithin), surfactants (e.g. sodium lauryl sulphate, polysorbate), dispersants (e.g. xanthan gum). For the preparation of the coat protecting the active ingredient from light, common pharmaceutically acceptable pigments (e.g. iron oxides, talc, aluminium-based compounds, titanium dioxide) or other suitable natural and synthetic dyes, stains and pigments can be advantageously used. The tablet cores can be preferably coated with a functional coat with 2 to 6% weight increase.

The final product in the form of capsules or tablets can be further packed in a protective blister pack or a bottle. If necessary, the protective pack can be fitted with a desiccant, oxygen absorber or a protective inert atmosphere (e.g. nitrogen).

Solubility of ibrutinib free base is pH dependent (SPC of the preparation Imbruvica® 140 hard gelatin capsules). For C of ibrutinib has been found to exhibit a three times higher dissolution rate in the acidic stomach pH. The dissolution rate of ibrutinib form C measured using the method of dissolution from a disk at pH = 2 is 0.052 mg·min⁻¹·cm⁻². The dissolution rate of ibrutinib form A measured at the identical conditions is 0.017 mg·min⁻¹·cm⁻².

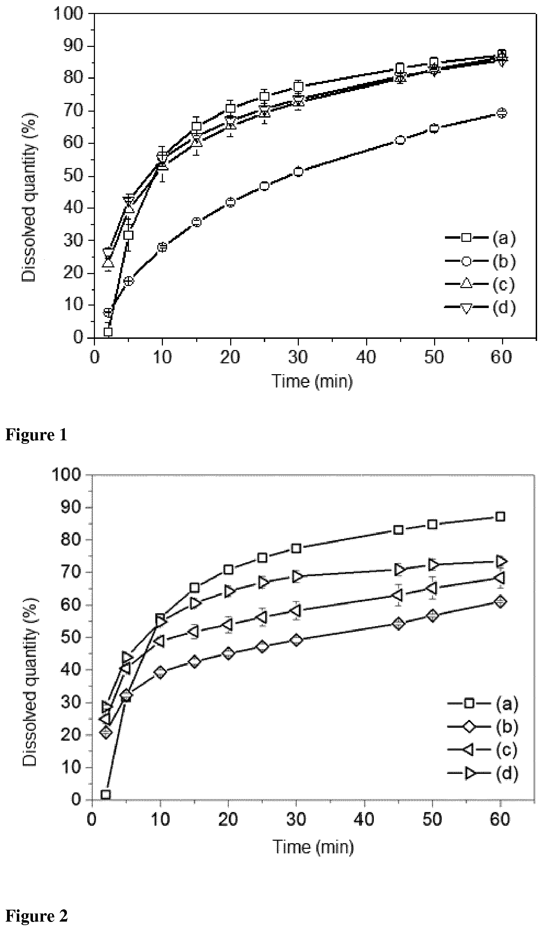

Due to the low solubility of form A of ibrutinib, within the preparation of Imbruvica®, the solubility of this drug form required optimization. For these reasons, a pilot pharmacokinetic animal study has been conducted to study the influence of micronization of form A of ibrutinib and the contents of various surfactants in the pharmaceutical composition. Based on this study, a size of ibrutinib particles and a type and quantity of the wetting agent for the pharmaceutical composition were selected. In the final product, the active ingredient is present in a micronized state and its micronization has an influence on bioavailability of the final product. The formulation process of the product Imbruvica® comprises mixing, dry granulation, grinding, encapsulation and packing (SPC of the preparation Imbruvica® 140 hard gelatin capsules). What has been surprisingly found out is that thanks to higher solubility of form C of ibrutinib there is no need for a surfactant (wetting agent) to be present in pharmaceutical compositions according to the present invention and the size of particles of the active ingredient does not need to be adapted by micronization either. While maintaining the same dose of the active ingredient, the same solubility and thus bioavailability was achieved in a dissolution experiment as in the case of the original composition prepared according to the patent application 2013/184572 (Fig. 1). In the case of a formulation comprising form C of ibrutinib, the presence of the surfactant sodium lauryl sulphate has been surprisingly found to have a negative influence on solubility of this form (Fig. 2).

In the patent application WO 2013/184572, in Example 10, which relates to a pharmaceutical composition in the form of capsules, the content of the surfactant (sodium lauryl sulphate) of 4.2% and 6.1% by weightis specified for the 140 mg dose of ibrutinib. The typical content of this surfactant varies in the range of 0.2-1.5% by weight in solid drug forma (The Handbook of Pharmaceutical Excipients, 6th editi*on).* With respect to the daily dose of the product Imbruvica®, which amounts to 560 mg of ibrutinib a day, the amount of the used sodium lauryl sulphate corresponds to 56 mg a day. Sodium lauryl sulphate (SLS) is a material of medium toxicity exhibiting acute toxicity on contact with the skin, eyes, mucous membranes, upper airways and stomach. The toxicity of SLS mainly results from the characteristics of this surfactant as it may cause disturbing of cell walls, releasing of cytokine and changes of protein conformation. Sensitivity to SLS depends on the formulation type (and the influence of other excipients), the amount of SLS, place of effect, exposure time, patient population and preparation process (*Background review for sodium lauryl sulfate used as an excipient, EMA*). The absence of the wetting agent in the composition according to the present invention has a positive influence on stomach irritation and the occurrence of some side effects of the drug Imbruvica®. The most common side effects of this drug comprise diarrhoea, fatigue, muscle and bone pain, bruise formation, nausea, infection of the upper airways and rash (*Imbruvica*® *label FDA).*

Within the use of the input crystalline ibrutinib free base (form C), four novel polymorph forms of ibrutinib (form 1 to form 4) could be prepared within the study of polymorphous behaviour.

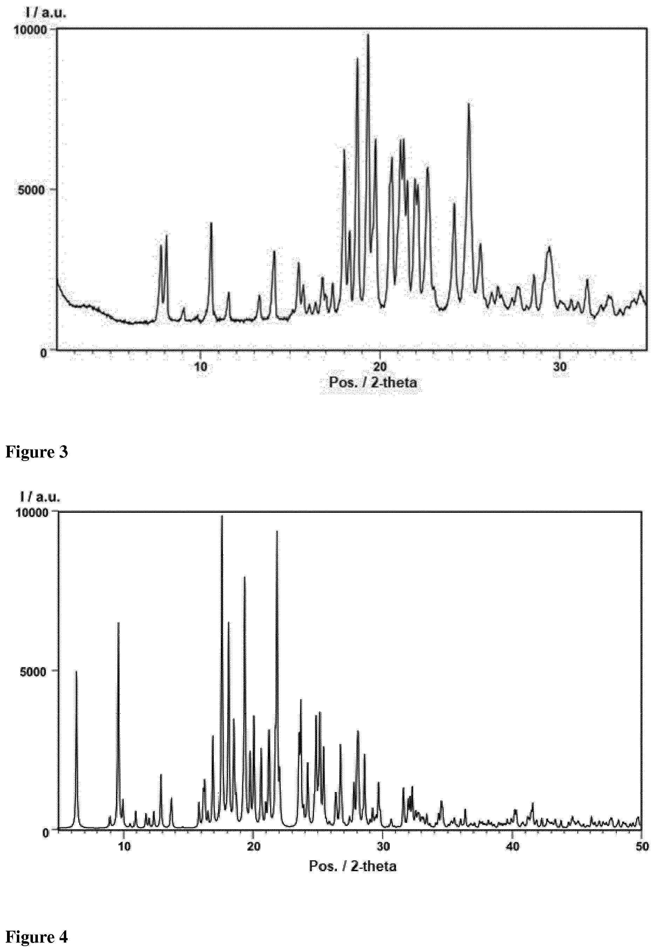

Crystalline form 1 of ibrutinib free base (prepared according to Example 1) is characterized by the reflections presented in **Table 1.** Table 1 comprises reflections whose relative intensity value is higher than 1%. The characteristic diffraction peaks of crystalline form 1 of ibrutinib free base according to the present invention with the use of CuKa radiation are: 8.11; 10.61; 18.73; 19.34; 21.14; 24.92; ± 0.2° 2-theta. The X-ray powder pattern is shown in **Fig. 3****.**

**Table 1**

| Position [°2Th.] | Interplanar distance [Å] [Å]=0.1nm | Rel. intensity [%] |
|---|---|---|
| 7.80 | 11.33 | 26.59 |
| 8.11 | 10.90 | 30.13 |
| 9.07 | 9.75 | 4.91 |
| 9.77 | 9.06 | 1.71 |
| 10.61 | 8.34 | 35.26 |
| 11.56 | 7.65 | 10.65 |
| 13.29 | 6.66 | 9.16 |
| 14.12 | 6.27 | 24.85 |
| 15.47 | 5.73 | 21.13 |
| 15.72 | 5.64 | 13.02 |
| 16.07 | 5.52 | 6.04 |
| 16.41 | 5.40 | 7.01 |
| 16.80 | 5.28 | 15.38 |
| 16.99 | 5.22 | 9.21 |
| 17.35 | 5.11 | 13.77 |
| 18.01 | 4.93 | 58.77 |
| 18.30 | 4.85 | 31.62 |
| 18.73 | 4.74 | 91.04 |
| 19.34 | 4.59 | 100.00 |
| 19.75 | 4.50 | 63.74 |
| 20.53 | 4.33 | 47.19 |
| 20.67 | 4.30 | 52.74 |
| 21.14 | 4.20 | 63.52 |
| 21.32 | 4.17 | 63.50 |
| 21.53 | 4.13 | 48.71 |
| 21.93 | 4.05 | 49.06 |
| 22.12 | 4.02 | 47.36 |
| 22.62 | 3.93 | 53.84 |
| 23.04 | 3.86 | 11.24 |
| 24.13 | 3.69 | 41.48 |
| 24.92 | 3.57 | 76.22 |
| 25.59 | 3.48 | 26.95 |
| 26.22 | 3.40 | 10.42 |
| 26.56 | 3.36 | 12.29 |
| 27.34 | 3.26 | 8.49 |
| 27.71 | 3.22 | 12.03 |
| 28.58 | 3.12 | 14.80 |
| 29.45 | 3.03 | 25.24 |
| 30.01 | 2.98 | 7.31 |
| 30.64 | 2.92 | 7.92 |
| 31.03 | 2.88 | 7.05 |
| 31.54 | 2.84 | 15.03 |
| 32.27 | 2.77 | 5.72 |
| 32.69 | 2.74 | 9.01 |
| 32.89 | 2.72 | 8.36 |
| 33.36 | 2.69 | 4.41 |
| 34.48 | 2.60 | 10.47 |

Crystalline form 2 of ibrutinib free base (prepared according to Example 2) is characterized by the reflections presented in **Table 2.** Table 2 comprises reflections whose relative intensity value is higher than 1%. The characteristic diffraction peaks of crystalline form 2 of ibrutinib free base according to the present invention with the use of CuKα radiation are: 6.49; 9.69; 17.68; 18.21; 19.44; 21.95 ± 0.2° 2-theta. The X-ray powder pattern is shown in **Fig. 4****.**

**Table 2**

| Position [°2Th.] | Interplanar distance [Å] [Å]=0.1nm | Rel. intensity [%] |
|---|---|---|
| 6.49 | 13.62 | 50.56 |
| 9.05 | 9.76 | 3.90 |
| 9.69 | 9.12 | 66.69 |
| 10.04 | 8.80 | 9.31 |
| 10.61 | 8.33 | 1.51 |
| 11.02 | 8.02 | 5.50 |
| 11.84 | 7.47 | 4.68 |
| 12.09 | 7.32 | 3.34 |
| 12.43 | 7.11 | 5.48 |
| 12.99 | 6.81 | 17.39 |
| 13.80 | 6.41 | 9.63 |
| 14.67 | 6.03 | 0.56 |
| 15.94 | 5.56 | 8.49 |
| 16.25 | 5.45 | 12.30 |
| 16.38 | 5.41 | 15.37 |
| 16.63 | 5.33 | 5.64 |
| 17.02 | 5.21 | 29.75 |
| 17.68 | 5.01 | 100.00 |
| 18.21 | 4.87 | 66.88 |
| 18.62 | 4.76 | 35.10 |
| 18.79 | 4.72 | 10.43 |
| 19.44 | 4.56 | 81.18 |
| 19.88 | 4.46 | 24.84 |
| 20.16 | 4.40 | 35.94 |
| 20.72 | 4.28 | 25.59 |
| 21.07 | 4.21 | 8.55 |
| 21.33 | 4.16 | 31.90 |
| 21.80 | 4.07 | 30.31 |
| 21.95 | 4.05 | 95.13 |
| 22.15 | 4.01 | 19.27 |
| 23.66 | 3.76 | 30.04 |
| 23.81 | 3.73 | 40.56 |
| 24.01 | 3.70 | 7.52 |
| 24.31 | 3.66 | 21.43 |
| 24.97 | 3.56 | 36.55 |
| 25.27 | 3.52 | 37.90 |
| 25.55 | 3.48 | 26.46 |
| 25.97 | 3.43 | 2.36 |
| 26.48 | 3.36 | 11.21 |
| 26.82 | 3.32 | 27.21 |
| 27.53 | 3.24 | 3.80 |
| 27.88 | 3.20 | 14.72 |
| 28.20 | 3.16 | 31.20 |
| 28.70 | 3.11 | 23.71 |
| 29.12 | 3.06 | 2.95 |
| 29.32 | 3.04 | 6.54 |
| 29.77 | 3.00 | 14.83 |
| 29.92 | 2.98 | 5.48 |
| 30.75 | 2.91 | 3.01 |
| 31.72 | 2.82 | 12.81 |

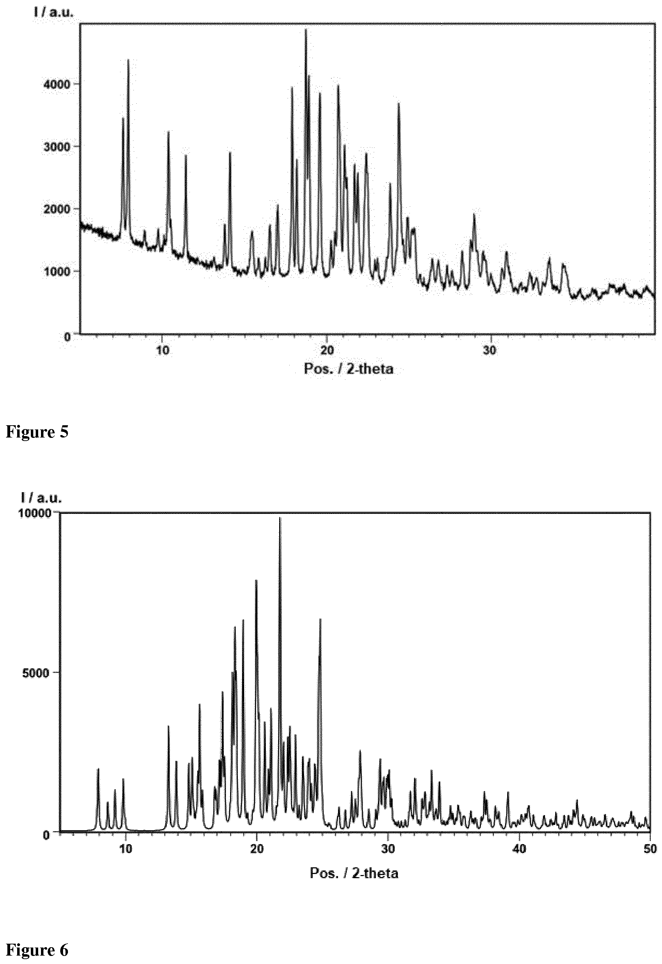

Crystalline form 3 of ibrutinib free base (prepared according to Example 3) is characterized by the reflections presented in **Table 3.** Table 3 comprises reflections whose relative intensity value is higher than 1%. The characteristic diffraction peaks of crystalline form 3 of ubrutinib free base according to the present invention with the use of CuKα radiation are: 7.92; 17.97; 18.71; 18.89; 20.66; 24.36 ± 0.2° 2-theta. The X-ray powder pattern is shown in **Fig. 5****.**

**Table 3**

| Position [°2Th.] | Interplanar distance [Å] [Å]=0.1nm | Rel. intensity [%] |
|---|---|---|
| 7.60 | 11.63 | 49.59 |
| 7.92 | 11.17 | 72.81 |
| 8.91 | 9.92 | 6.03 |
| 9.74 | 9.08 | 8.12 |
| 10.36 | 8.54 | 50.11 |
| 10.50 | 8.42 | 13.89 |
| 11.42 | 7.75 | 42.25 |
| 13.15 | 6.73 | 3.12 |
| 13.76 | 6.44 | 18.04 |
| 14.09 | 6.29 | 47.95 |
| 15.46 | 5.73 | 15.80 |
| 15.84 | 5.59 | 5.92 |
| 16.25 | 5.46 | 6.30 |
| 16.51 | 5.37 | 19.54 |
| 16.99 | 5.22 | 28.13 |
| 17.87 | 4.96 | 75.92 |
| 18.15 | 4.89 | 44.34 |
| 18.71 | 4.74 | 100.00 |
| 18.89 | 4.70 | 81.40 |
| 19.57 | 4.54 | 74.75 |
| 20.24 | 4.39 | 15.31 |
| 20.46 | 4.34 | 17.29 |
| 20.66 | 4.30 | 76.03 |
| 20.82 | 4.27 | 36.21 |
| 21.06 | 4.22 | 54.14 |
| 21.20 | 4.19 | 40.89 |
| 21.67 | 4.10 | 46.59 |
| 21.87 | 4.06 | 43.20 |
| 22.40 | 3.97 | 51.64 |
| 22.91 | 3.88 | 7.63 |
| 23.09 | 3.85 | 8.46 |
| 23.83 | 3.73 | 38.79 |
| 24.36 | 3.65 | 71.41 |
| 24.93 | 3.57 | 26.58 |
| 25.34 | 3.51 | 21.50 |
| 25.66 | 3.47 | 3.91 |
| 26.41 | 3.38 | 10.50 |
| 26.78 | 3.33 | 9.87 |
| 27.27 | 3.27 | 7.62 |
| 27.58 | 3.23 | 5.74 |
| 28.21 | 3.16 | 14.84 |
| 28.72 | 3.11 | 18.79 |
| 28.93 | 3.09 | 28.67 |
| 29.48 | 3.03 | 15.08 |
| 29.65 | 3.01 | 11.25 |
| 29.97 | 2.98 | 5.86 |
| 30.60 | 2.92 | 7.84 |
| 30.90 | 2.89 | 15.16 |
| 31.78 | 2.82 | 2.65 |
| 32.33 | 2.77 | 7.55 |
| 32.77 | 2.73 | 5.48 |
| 33.13 | 2.70 | 4.40 |
| 33.56 | 2.67 | 13.42 |
| 34.32 | 2.61 | 11.89 |
| 35.37 | 2.54 | 1.91 |
| 36.19 | 2.48 | 1.89 |
| 37.32 | 2.41 | 3.78 |
| 38.07 | 2.36 | 3.82 |
| 39.47 | 2.28 | 3.35 |

Crystalline form 4 of ibrutinib free base (prepared according to Example 4) is characterized by the reflections presented in **Table 4.** Table 4 comprises reflections whose relative intensity value is higher than 1%. The characteristic diffraction peaks of crystalline form 4 of ibrutinib free base according to the present invention with the use of CuKα radiation are: 15.65; 18.29; 18.94; 19.94; 21.73; 24.83 ± 0.2° 2-theta. The X-ray powder pattern is shown in **Fig. 6****.**

**Table 4**

| Position [°2Th.] | Interplanar distance [Å] [Å]=0.1nm | Rel. intensity [%] |
|---|---|---|
| 7.91 | 11.16 | 20.08 |
| 8.65 | 10.22 | 9.25 |
| 9.17 | 9.64 | 13.37 |
| 9.80 | 9.02 | 16.46 |
| 11.42 | 7.74 | 0.10 |
| 13.26 | 6.67 | 32.96 |
| 13.85 | 6.39 | 22.41 |
| 14.81 | 5.98 | 21.63 |
| 15.08 | 5.87 | 23.16 |
| 15.49 | 5.72 | 18.28 |
| 15.65 | 5.66 | 40.05 |
| 15.86 | 5.58 | 12.85 |
| 16.79 | 5.28 | 13.57 |
| 16.92 | 5.23 | 13.07 |
| 17.16 | 5.16 | 22.48 |
| 17.34 | 5.11 | 43.90 |
| 17.50 | 5.06 | 23.11 |
| 18.12 | 4.89 | 49.68 |
| 18.29 | 4.85 | 63.28 |
| 18.41 | 4.82 | 47.94 |
| 18.94 | 4.68 | 66.26 |
| 19.27 | 4.60 | 5.72 |
| 19.67 | 4.51 | 6.37 |
| 19.94 | 4.45 | 77.89 |
| 20.15 | 4.40 | 35.38 |
| 20.60 | 4.31 | 34.18 |
| 20.88 | 4.25 | 19.41 |
| 21.06 | 4.21 | 38.46 |
| 21.47 | 4.14 | 7.58 |
| 21.73 | 4.09 | 100.00 |
| 22.00 | 4.04 | 27.83 |
| 22.33 | 3.98 | 29.81 |
| 22.50 | 3.95 | 32.82 |
| 22.92 | 3.88 | 30.15 |
| 23.20 | 3.83 | 8.10 |
| 23.49 | 3.78 | 23.12 |
| 23.88 | 3.72 | 20.53 |
| 23.99 | 3.71 | 22.33 |
| 24.15 | 3.68 | 14.87 |
| 24.40 | 3.65 | 20.93 |
| 24.71 | 3.60 | 52.56 |
| 24.83 | 3.58 | 64.48 |
| 25.48 | 3.49 | 2.42 |
| 26.12 | 3.41 | 4.08 |
| 26.25 | 3.39 | 7.48 |
| 26.72 | 3.33 | 6.56 |
| 27.22 | 3.27 | 12.41 |
| 27.48 | 3.24 | 9.98 |
| 27.87 | 3.20 | 25.12 |
| 28.52 | 3.13 | 6.83 |
| 29.07 | 3.07 | 6.67 |
| 29.40 | 3.04 | 22.74 |
| 29.69 | 3.01 | 16.43 |
| 29.87 | 2.99 | 15.90 |

The pharmaceutical compositions according to the present invention can be used for the preparation of solid drug forms, e.g. tablets or capsules and can comprise at least one excipient from the group of fillers (e.g. lactose, microcrystalline cellulose - Avicel) in the quantity of 20 to 60% by weight, binders (e.g. povidone, hydroxypropyl cellulose) in the quantity of 1 to 35% by weight, disintegrants (e.g. sodium salt of croscarmellose) in the quantity of 3 to 10% by weight, lubricants (e.g. magnesium stearate, colloidal silicon dioxide) in the quantity of 0.3 to 3% by weight. The pharmaceutical compositions according to the present invention do not comprise a surfactant. Ibrutinib of form C can be mixed with the said excipients, screened through a sieve, and the resulting mixture can be compacted by roller compaction, wet granulation or tabletted. The granulate can be filled into capsules or mixed with other added excipients and tabletted. The tablets can be further coated with common or functional coats consisting e.g. of polyvinyl alcohol, polyethylene glycol or hydroxypropyl methyl cellulose in the quantity of 50 to 70% by weight, a plasticizer in the quantity of 3 to 14% by weight, a dye in the quantity of 15 to 25% by weight, a pigment in the quantity of 0.1 to 1.0% by weight and a glidant in the quantity of 8.5 to 10% by weight.

The final product in the form of capsules or tablets can be further packed in a protective package, in a protective package with a desiccant, in a protective package with an oxygen absorbent, or in a protective package with a protective inert atmosphere.

The invention is clarified in more detail in the embodiment examples below. These examples, which illustrate preparation of solid forms of ibrutinib and pharmaceutical compositions with ibrutinib of form C, exclusively have an illustrative character and do not restrict the scope of the invention in any respect.

### Experimental part

### Single-crystal X-ray diffraction

The diffractograms were obtained using a Gemini single-crystal diffractometer by Oxford Diffraction, used CuKα radiation (λ=1.542 Å) with an Atlas CCD detector or using a SuperNova monocrystal Diffractometer by Rigaku Oxford Diffraction, used CuKα radiation (λ=1.542 Å) with an Atlas S2 CCD detector. The analysis of single-crystal X-ray diffractions was conducted at the temperature of -153.15°C (Gemini) and -178.15°C (SuperNova). The measured data were processed and evaluated in the program CrysAlisPro, Rigaku Oxford Diffraction, 2015, version 1.171.38.41q. The SCALE3 ABSPACK scaling algorithm was used to carry out the empirical correction of absorptions. The crystal structures were dealt with using a direct method in a program (SIR92) and specified in the CRYSTALS 14.40b24 and Jana2006 programs. All the atoms except hydrogen atom were specified in an anisotropic way. Powder X-ray diffraction was calculated with the use of Mercury software (version 3.3).

### Dissolution rate determination using the method of dissolution from powder

The dissolution rate of all the tested forms was measured using a Sotax AT-7 dissolution device with the use of paddles (USP 2). The dissolution was conducted in 900 ml of a solution having pH 2.0 (10 mM HCl) at the constant rate of 75 rpm until minute 45 of the experiment and at the increased rate of 150 rpm until minute 60. Samples were taken in 5-minute intervals and the concentration of dissolved ibrutinib was determined with the use of a Jena Analytik UV/Vis spectrophotometer at the wavelength of 300 nm in 5 mm cuvettes.

### Examples

The crystalline free base of ibrutinib form A was prepared in accordance with the procedure disclosed in the patent application WO 2013/184572. The prepared ibrutinib was treated by grinding.

The crystalline free base of ibrutinib form C was prepared in accordance with the procedure disclosed in the patent application WO 2017/174044.

Examples of pharmaceutical compositions are presented in table 5 and table 6.

### Example 1

### Preparation of crystalline ibrutinib (form 1)

Crystalline free base of 1-[(3*R*)-3-[4-Amino-3-(4-phenoxyphenyl)-1*H*-pyrazolo[3,4-d]pyrimidin-1-yl]piperidin-1-yl]prop-2-en-1-one (200 mg, form C) was suspended in 1 ml of a saturated solution of ibrutinib in bromobenzene. The obtained suspension was shaken at the laboratory temperature for 48 h and subsequently the solid fraction was filtered. The resulting product was dried at a reduced pressure of 200 mbar at the temperature of 40°C for 12 h. The X-ray powder pattern is in Fig. 3.

### Example 2

### Preparation of crystalline ibrutinib (form 2)

Crystalline free base of 1-[(3*R*)-3-[4-Amino-3-(4-phenoxyphenyl)-1H-pyrazolo[3,4-d]pyrimidin-1-yl]piperidin-1-yl]prop-2-en-1-one (100 mg, form C) was dissolved in 1 ml of fluorobenzene. The obtained solution was then filtered and left to slowly dry out during 2 weeks. Grown crystals were removed from the remaining solution and measured using a single-crystal diffractometer. The X-ray powder pattern is in Fig. 4.

### Example 3

### Preparation of crystalline ibrutinib (form 3)

Crystalline free base of 1-[(3*R*)-3-[4-Amino-3-(4-phenoxyphenyl)-1H-pyrazolo[3,4-d]pyrimidin-1-yl]piperidin-1-yl]prop-2-en-1-one (200 mg, form C) was suspended in 1 ml of a saturated solution of ibrutinib in iodobenzene. The obtained suspension was shaken at the laboratory temperature for 48 h and subsequently the solid fraction was filtered. The resulting product was dried at a reduced pressure of 200 mbar at the temperature of 40°C for 12 h. The X-ray powder pattern is in Fig. 5.

### Example 4

### Preparation of crystalline ibrutinib (form 4)

Crystalline free base of 1-[(3*R*)-3-[4-Amino-3-(4-phenoxyphenyl)-1H-pyrazolo[3,4-d]pyrimidin-1-yl]piperidin-1-yl]prop-2-en-1-one (100 mg, form C) was dissolved in 1 ml of *α,α,α*-trichlorotoluene. The obtained solution was then filtered and left to slowly dry out during 2 weeks. Grown crystals were removed from the remaining solution and measured using a single-crystal diffractometer. The X-ray powder pattern is in Fig. 6.

### Example 5

To achieve better flow properties of the material, the prepared mixtures of ibrutinib and excipients (Table 5 and 6) are processed by briquetting using a StylOne machine with the use of round dies with the diameter of 20 mm. Manual filling of a die with 750 mg of the mixture of ibrutinib and excipients. These mixtures were briquetted using the pressure of 10 and 25 kN. The compaction product was obtained by subsequent screening through a 0.8 mm sieve.

### Example 6-8

Figures 1 and 2 show the dissolution curves of the treatment preparation *Imbruvica*®, prepared using a mixture of ibrutinib of form C with excipients and individual compaction products.

Examples of compositions are presented in the tables below.

**Table 5:**

| Mixture containing 140 of ibrutinib form C | | | | |
|---|---|---|---|---|
| Component | Example 6 quantity (mg) | quantity (%) | Example 7 quantity (mg) | quantity (%) |
| Ibrutinib (form C) | 140.0 | 42.5 | 140.0 | 42.5 |
| Avicel PH102 | 164.8 | 50.0 | 150.9 | 45.8 |
| sodium salt of croscarmellose | 23.1 | 7.0 | 23.1 | 7.0 |
| Sodium lauryl sulphate | - | - | 13.8 | 4.2 |
| Magnesium stearate | 1.6 | 0.5 | 1.65 | 0.5 |
| Mixture weight | 329.5 | | 329.5 | |

**Table 6:**

| Mixture containing 140 mg of ibrutinib form A - treatment preparation *Imbruvica*® | | |
|---|---|---|
| Component | Example 8 quantity (mg) | quantity (%) |
| Ibrutinib (form A) | 140.0 | 42.5 |
| Avicel PH102 | 150.9 | 45.8 |
| sodium salt of croscarmellose | 23.1 | 7.0 |
| sodium lauryl sulphate | 13.8 | 4.2 |
| Magnesium stearate | 1.65 | 0.5 |
| Mixture weight | 329.5 | |

### Example 9

A part of the prepared tablets was coated with a coloured coat with a tablet coating drum device. The composition of the coloured coat is presented in Table 7.

**Table 7:**

| Component | Component content (%) |
|---|---|
| Hypromellose | 63.3 |
| Polyethylene glycol | 6.7 |
| Titanium dioxide | 20.0 |
| Iron (III) oxide | 0.2 |
| Talc | 9.8 |

### Example 10

Using the method of dissolution from powder it was found out that thanks to higher solubility of form C of ibrutinib, there is no need for a surfactant (wetting agent) to be present in pharmaceutical compositions according to the present invention and the size of particles of the active ingredient does not need to be adapted by micronization either. Maintaining the same dose of the active ingredient, the same solubility was achieved in the dissolution experiment as with the original preparation Imbruvica® (Fig. 1). Further, in the case of the formulation of form C of ibrutinib, the presence of the surfactant sodium lauryl sulphate was found to have a negative influence on solubility of this form (Fig. 2).

## Claims

1. A pharmaceutical composition, **characterized in that** it comprises crystalline form C of ibrutinib free base and at least one pharmaceutically acceptable excipient and it does not comprise a surfactant.

2. The pharmaceutical composition according to claim 1, **characterized in that** the at least one pharmaceutically acceptable excipient is selected from a group that consists of fillers, binders, disintegrants and lubricants.

3. The pharmaceutical composition according to claim 1 and 2, **characterized in that** it comprises:
crystalline form C of ibrutinib free base in the quantity of 30 to 50% by weight;
filler in the quantity of 20 to 60% by weight;
other excipients up to 100% of the content of the composition, which are a disintegrant, binder and/or lubricant.

4. The pharmaceutical composition according to claim 1 to 3, **characterized in that** it is in a capsule form.

5. The pharmaceutical composition according to claim 1 to 3, **characterized in that** it is in a tablet form.

6. The pharmaceutical composition according to claim 5, **characterized in that** the tablet further comprises a film coat.

7. The pharmaceutical composition according to claim 6, **characterized in that** the film coat comprises:
binder in the quantity of 50 to 70% by weight;
plasticizer in the quantity of 3 to 14% by weight;
dye in the quantity of 15 to 25% by weight;
pigment in the quantity of 0.1 to 1.0% by weight;
glidant in the quantity of 8.5 to 10% by weight.

8. The pharmaceutical compositions according to claims 1 to 7, **characterized in that** it is packed in a protective package, in a protective package with a desiccant, in a protective package with an oxygen absorbent, or in a protective package with a protective inert atmosphere.

9. A pharmaceutical composition, **characterized in that** it comprises:
140 mg of crystalline form C of ibrutinib free base;
165 mg of microcrystalline cellulose;
23 mg of sodium salt of croscarmellose;
2 mg of magnesium stearate.
